# EUROPEAN PATENT SPECIFICATION

(11) **EP 1 443 968 B1**
(45) Date of publication and mention of the grant of the patent: **18.06.2008**
(21) Application number: 02780695.9
(22) Date of filing: 14.11.2002
(51) Int. Cl.: A61K 47/00

(54) **EDIBLE FILM**
ESSBARE FOLIE
FILM COMESTIBLE

(30) Priority: 16.11.2001 US 333228 P
(43) Date of publication of application: 11.08.2004
(73) Proprietor: Givaudan SA, 1214 Vernier (CH)
(72) Inventor: VIRGALITTO, Margaret, T., Dayton, OH (US); ZHANG, Jing, Loveland, OH 45150 (US)
(74) Representative: McStea, John Anthony
(86) International application number: PCT/US2002/036766
(87) International publication number: WO 2003/043659

(56) References cited:
- WO-A-92/03223
- US-A- 4 205 060
- US-A- 5 102 668
- PATENT ABSTRACTS OF JAPAN vol. 2000, no. 15, 6 April 2001 (2001-04-06) & JP 2000 342186 A (YUUSU KK), 12 December 2000 (2000-12-12)
- PATENT ABSTRACTS OF JAPAN vol. 1999, no. 03, 31 March 1999 (1999-03-31) & JP 10 327770 A (HATATE YASUO; KOUNO SHIGENOBU), 15 December 1998 (1998-12-15)

## Description

The invention is concerned with edible film and in particular edible film that contains an active substance, for example a flavourant, fragrance, pharmaceutical or nutraceutical, or mixtures thereof.

Edible film is useful in the food and food service industries as an easy to use, cheap and convenient means of flavouring food products. It is also useful to form articles for placing in the oral cavity to administer thereto a flavourant or breath-freshening agent. Further use is made of edible films in the delivery of pharmacuetical or nutraceutical active ingredients. This form of administration of such materials is particularly useful for patient populations such as infants or geriatrics that may be unable, or find it difficult, to swallow conventional oral dosage forms.

Examples of edible films containing active ingredients found in the literature are provided by WO 00/18635 and US 5,948,430. These references are concerned with edible films that incorporate either breath-freshening agents or pharmaceutical agents, together or optionally with flavourants. Both references teach only the simple admixture of active oils into the bulk film-forming materials to form homogenous mixtures that may be cast into films.

Through experience, the applicant has found that interactions between active agent and film-formers results in the modulation of a film's physical properties, and also influences the properties of the active agent, i.e. either the active agent is degraded, or its release characteristics are influenced adversely by the film formers, or it cannot be added in large enough amounts to provide an optimal effect. Unfortunately, such approaches to edible film formation represent a compromise between the physical properties of the film, such as storage stability and ease of handling, and the delivery profile of the active ingredient.

These problems are particularly pronounced in the case of delivery of flavourants or fragrance materials. Flavourant and fragrance materials almost never consist of individual ingredients. Typically they are composed of a complex mixture of ingredients of disparate physicochemical properties such as volatility, solubility and chemical reactivity. As such, the components may evaporate or degrade at different rates and to different extents. Furthermore, the film-forming materials may exert a chromatographic effect on the flavour or fragrance compositions, that is, they may preferentially bind or trap certain ingredients of the composition such that these ingredients cannot be fully expressed from the film during use. Perfumers and flavourists seek to present balanced flavour or fragrance accords. The preferential loss or degradation of certain ingredients to the film, may create an imbalance that renders the flavourant or fragrance practically useless. A specially designed flavour or fragrance composition is of no value if the delivery vehicle into which it is incorporated cannot deliver it with fidelity.

Active agents in general, but specifically flavourants or fragrances are therefore particularly difficult to formulate according to the conventional teaching of admixing actives directly into film-forming materials.

The failings of the conventional teaching are not limited to the poor delivery or performance of the active agents. The addition of active agents directly to film forming ingredients may influence the film's physical properties or may introduce process constraints in the film's manufacture that may add complexity, duration and cost to the film-making process. For example, large active loading, particulary if the active is a liquid or in solution, may render films sticky and self-adhering, and of poor mechanical stability. As a result, the formulator's latitude in his choice of film-forming ingredients and active agents may be affected. Still further, drying temperatures during manufacture of the films may have to be modified to ensure that volatile or unstable actives are not lost through evaporation or degradation.

In summary, the formation of edible films poses a considerable technical challenge that the prior art does not adequately address. There remains a need for edible film that can reliably retain an active agent with a high loading, in particular a flavour or fragrance material during processing and storage and which is mechanically robust to withstand long periods of storage, and handling. It is also desirable to provide a film with the aforementioned attributes and which also disintegrates, disperses or dissolves rapidly to release its active agent on demand, for example when placed on a food product during cooking, or when placed directly in or on the human body, e.g. in the oral cavity, without causing adverse mouth feel.

Applicant has now found that edible film that addresses all of these concerns can be obtained when all, or substantially all of the active agent is sequestered from the film-forming materials by means of a suitable encapsulation means.

### Summary of the Invention

Accordingly, the invention provides in a first aspect an edible film wherein said film comprises a hydrocolloid film-forming material having dispersed therein a plurality of microcapsules containing an active agent.

The invention is possessed of many advantages related both to film stability and active agent delivery. The edible film displays good oxidative stability and product shelf-life, preserves active agent integrity during thermal processing, displays hydrolytic stability during processing and storage, overcomes undesirable binding interactions between active agent and the film-forming ingredients, facilitates mixing of incompatible ingredients, masks undesirable odour or taste properties, and provides the formulator with a greater latitude to design specific controlled release properties for the particular active agent to be delivered.

All manner of technical effects relating to delivery of active agent can be achieved using microcapsules. In the simplest embodiment, a single active ingredient is employed in the microcapsules that is released in a time dependent manner as a result of diffusion or in response to an exogenous stimulus leading to the degradation of the microcapsule, such as heat or moisture in a cooking process, moisture from the oral cavity, or from mechanical disruption , e.g. by mastication. However, the present invention also covers microcapsules that are multifunctional, that is, there may be different populations of microcapsules containing different active agents selected from pharmaceutials, nutraceuticals, flavourants and fragrances, or mixtures thereof.

Furthermore, not only can the populations of microcapsules be differentiated in terms of the nature of the active agent contained therein, the invention also provides that the microcapsules may comprise different populations in terms of the nature of the microencapsulating medium, thereby to influence the release kinetics of the active ingredients contained in different microcapsule populations.

The present invention therefore provides the formulator with considerable latitude to effect release of different active agents on demand, in a time-dependant manner. This can be particularly advantageous in relation to delivery of flavours or fragrances for the reasons enumerated above. The flavourist or perfumer has greater latitude to employ the range of his ingredients palette with neither concern for the effects certain ingredients shall have the on film's properties, nor concern for possible ingredient loss, through evaporation or degradation, or due to chromatographic effects.

By sequestering active agent from the film-forming material in this way, the invention also enables high loading of active agent without causing any deleterious effects on film stability. However, because the bulk of the active agent may be contained in a microencapsulated form, some active agent may also be incorporated in the bulk film to add a further dimension to active agent release, although the skilled person will , appreciate that the amount that may be added is limited by considerations of film stability and active agent release, and will take precautions to this effect.

Microcapsules may be employed to contain certain non-active agents such as colourants. It is clear that in consumer products, colour and patterns of colours help consumers identify with a certain product or brand of product. However, it is technically difficult to introduce colours, and in particular, combinations of colours into an edible film without colours leaching out of their assigned configurations during manufacture and during prolonged periods of storage. Employing pre-coloured populations of microcapsules provides a simple means of colouring films effectively, even with intricate designs. Furthermore, because they are encapsulated, the colours display a considerably reduced tendency to leach or diffuse over time.

Finally, microcapsules can be used to added additional visual impact to the edible film of the present invention by using microcapsule populations having different diameters to give an impression of particulate matter in the fiilm.

The edible film of the present invention may contain other opitional ingredients that may confer additional benefits or properties on the film.

One or more disintegrants may be employed. Disintegrants are water-soluble materials that dissolve rapidly upon contact with moisture causing the film structure to breakdown and disintegrate. The nature of the disintegrant, and the amount employed in the film will depend on how fast one requires the film to disintegrate and dissolve, and also on the nature of the film-forming material.

Plasticisers may be employed to influence mechanical properties thereby to ensure that the film is soft, not easily friable and to ensure ease of handling thereof.

Emulsifiers may be employed to facilitate ease of manufacture of the films. The films may be formed of materials that are immiscible or not easily mixed without some form of intervention. Typically, if a film is cast onto a release paper for ease of storage, it is often the case that a continuous film cannot be formed without the intervention of emulsifiers. Films may be blotchy or streaky, or may even contain holes without the use of emulsifiers.

Other opitional ingredients may include perservatives and anti-microbial agents as are commonly employed in foodstuffs, nutrient formulations and pharmaceutical preparations.

### Detailed Description of the Invention

The hydrocolloid film forming materials may be any of those hydrocolloids known for their film-forming properties in the food industry, or the pharmaceutical industry. Any film-forming material that is capable of rapidly hydrating, and dispersing or dissolving in water may be employed. Preferred film-forming materials may be based in general on polymeric carbohydrates, or water-soluble polypeptides, and include one or more materials selected from starches; chemically or physically modified starches; alginates, e.g. sodium alginate; pectins; tragacantha, acacia gum, gum arabic; agar; carrageenan; gelatin; casein; soy protein; whey protein; cellulose polymers such as hydroxypropylmethyl cellulose, hydroxyethyl cellulose or hydroxypropyl cellulose, or mixtures thereof; wheat glutten, furcellaran, locust bean gums, chitosan, xanthan gum, gellan gum, pullalan and mixtures thereof. Synthetic polymers, soluble or swellable in water, may also be employed and examples thereof include polyvinylpyrrolidone, polyvinylalcohol, polyacrylic acid, or polyacrylamide.

The materials are chosen for their ability to rapidly hydrate, and disperse or dissolve on a substrate to which they are applied, or in the oral cavity. Preferred materials dissolve or disperse in the oral cavity, or on food to which they are applied within about 20 seconds to release the active agent contained in the film, and present the encapsulated active agent thereby to deliver a sustained release of active agent.

The materials also are chosen for their ability to form films when dry or substantially dry with the requisite mechanical strength that enables them to be handled and manipulated during manufacture, storage and use.

In addition to the properties described above, particularly preferred film-formers are those that can be incorporated in high amounts in a given volume of water in order that during their preparation, the edible film formulations contain as high solids content as possible, for example, in order to accelerate drying conditions. In this regard, alginates are particularly preferred film-forming materials.

The hydrocolloid film-forming material may be employed in varying amounts depending on the nature of the material, the particular film-forming conditions employed, the desired properties of the edible film, and the nature of the other ingredients employed in the film. For most purposes however, high amounts of film-forming material is desirable, e.g. from 50 to 90%, more particularly 50 to 80% by weight of the total solids of the film-forming composition.

All manner of encapsulation technologies may be applied in the present invention. The particular encapsulating medium used will depend upon the nature of the material to be encapsulated, the desired release kinetics and release profile. Apprised of these factors, the skilled person would not have to resort to inventive activity to select a suitable encapsulating medium to achieve a desired result.

Encapsulation techniques suitable in the present invention include spray-drying, complex coacervation, phase separation techniques (both aqueous and organic phase separation), cyclodextrin molecular encapsulation, yeast-cell encapsulation, in-situ polymerisation, coating, and extrusion.

Spray drying techniques are well known in the art, and can be used by the skilled person to form suitable microcapsules for use in the present invention. In a typical spray-drying technique, an active agent, usually in the form of an oil or in non-aqueous solution is dispersed in an aqeuous phase containing film-forming agent to form an emulsion that is fed into a drier through a nozzle that disperses the emulsion into small droplets. The drying conditions are chosen depending on a number of factors relating to desired product characteristics and particle size desired. All manner of film-forming agents may be employed, for example the film-forming carbohydrates, polypeptides and synthetic polymers recited above as being useful edible film forming materials can be employed .

Coacervation is a technique well known in the art and involves the steps of forming a hydrophobic core material containing active agent and emulsifying this in a charged, water-soluble polymer solution having the properties of a protective colloid. Thereafter, an oppositely charged hydrophilic colloid solution is added thereto. Process conditions such as colloid concentration, pH and temperature are controlled to induce phase separation (coacervation) to precipate a colloid-rich coating of the polymer onto the hydrophobic active-containing core to form a microcapsule wall. The wall is thereafter hardened and insolubilised by crosslinking using suitable cross-linkers such as aldehydes, e.g formaldehyde. Materials for use in the capsule wall are well known in the art and include proteins such as gelatin, or film-forming carbohydrates as aforementioned such as alginates.

Encapsulation by extrusion can proceed by making a melt of a matrix material, or a solution of matrix material and co-extruding this with an active agent, using a screw extrude or the like, before drying, or cooling, and grinding to form microcapsules. Matrix material may be formed of a hydrophilic and glassy material such as a water-soluble sugar or sugar mixture. Such matrices are typically impervious to moisture and oxidants and are useful to enapsulate oxidation- and moisture-sensitive active agents.

Alternatively, matrix materials may be hydrophobic, such as a vegetable fat, edible waxes, or film.forming carbohydrate, or even mixtures of hydrophobic and glassy-hydrophilic materials; the combinations of materials being selected to achieve a particularly desired delivery effect, having regard to the active agent.

Particles of active agent may also be coated with encapsulating media of any of the film-forming materials referred to herein above. Coating techniques may be used to coat particles, usually solid particles, of active agent, or even may be used to further coat encapsulated forms described herein above.

Coating may be carried out according to known techniques such as spray coating, pan coating, fluid bed coating, rotogranulator coating, annular jet coating, spinning disk coating, spray cooling, spray drying, filtermat drying, Multi Stage Drying (MSD) drum roll coating, freeze drying, and spray chilling.

The skilled person will appreciate that the particular technique used and the encapsulating material employed will depend upon the nature of the active agent to be encapsulated and the type of release characteristic that is sought to be achieved. For example when a flavouring agent is employed that contains a flavourant aldehyde it is preferred not to employ an encapsulating material that contains a polypeptide such as gelatin, as the aldehyde will act to crosslink the polypeptide over prolonged periods of time and this may effect the films ability to hydrate and dissolve, or disperse rapidly when placed, for example, in the mouth. Furthermore, if food acids are employed as active agent, encapsulating media are preferably selected from fatty substances such as edible waxes, and vegetable fats and the like, which materials efficiently encapsulate, and are not compromised by the presence of acids.

Notwithstanding the foregoing, the applicant has found that generally excellent encapsulation of many active agents, in particular flavourants can be achieved with microcapsules comprising an outer hydrogel shell surrounding a core material containing the flavour material. The hydrogel shell may be selected from those polypeptides, film-forming carbohydrates or synthetic polymers described above, e.g. gelatin, more particularly fish gelatin; and polyvinyl pyrollidone.

The core may consist of an active agent, e.g. a flavour material, or it may comprise flavour material and a carrier oil, for example an oil selected from a mineral oil, a vegetable oil, benzyl alcohol, or mixtures thereof. The microcapsules and the method of formation thereof are preferably those disclosed in US patent 6,045,835 which is incorporated herein by reference. Such microcapsules are commercially available under the trademark FLAVORBURST, from Givaudan.

The precise quantity of microcapsules added to the edible film formulation will depend on the loading of fragrance material therein. However, preferably microcapsules are added to achieve an active agent loading of 1 to 30 % based on dry weight of the composition, more preferably 1 to 10% dry weight. Depending on the type of encapsulation system employed and the nature of the active agent, it is possible to achieve loading from about 1 to 40% more preferably 10 to 25%.

Disintegrants used in the present invention are materials that may be added to the edible folm formulation to aid in the break-up of the film-forming material in a fluid environment. Preferred distinegrants may be selected from the water-soluble or water dispersible ingredients typically selected from the group consisting of a sugar, such as lactose, glucose, or mannose; a sugar alcohol, such as mannitol, sorbitol, xylitol, erythritol, lactitol, or maltitol; certain native starches or chemically or physically modified starches as known in the art, such as corn starch, potato starch, rice starch, tapioca starch, maize starch or sodium starch glycolate; synthetic polymers such as polyethylene glycol, polyvinylpyrrolidone, polyvinylalcohol, polyoxyethylene copolymers, polyoxypropylene copolymers, or polyethyleneoxide; cellulose-based materials like modified cellulose, crosscarmellose sodium and microcrystalline cellulose or a mixture of any of these compounds.

The modified starches are particularly preferred, for example tapioca starches or modified tapioca starch or combinations thereof.

The amount of disintegrant employed, if at all, depends on the nature of the film-forming material and the rate of disintegration that is required. Preferably, the disintegrant is employed in amounts of from 0 to 50% based on the dry weight of the formulation, more particularly 10 to 30% based on the dry weight of the formulation.

Active ingredients that may be delivered from an edible film according to the invention may be selected from pharmaceutical agents, nutraceutical agents, flavourants and other taste-modifying agents such as food acids, and fragrances. ,

The particular flavour ingredients employed depend on the end-use of the edible film. Flavour ingredients may be employed to impart a savoury taste to a food product. However, more preferably the flavour ingredients employed are used in films intended for breath-freshening applications or for confectionery or cosmetic products, or even to impart a pleasant taste, or taste.masking effect, to pharmaceutical or nutraceutical preparations.

Flavourants may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds.

Examples of suitable flavour components include without limitation 2-Methyl Pyrazine, Acetophenone Extra, Alcohol C6, Alcohol C8, Aldehyde C7 Heptylic, Aldehyde C8, Aldehyde C9, Allyl Caproate, Amyl Butyrate, Anisicaldhyde, Benzaldehyde, Benzyl Acetate, Benzyl Alcohol, Benzyl Butyrate, Benzyl Formate, Benzyl Iso Valerate, Benzyl Propionate, Butyl Acetate, Camphor, Cinnamic Aldehyde, Cis-3-Hexenol, Cis-3-Hexenyl Acetate, Cis-3-Hexenyl Formate, Cis-3-Hexenyl Propionate, Citronellal, Citronellol, Cuminic Aldehyde, Damascenone, Damascone Alpha, Damascone Beta, Diethyl Malonate, Dimethyl Anthranilate, Dimethyl Benzyl Carbinyl Acetate, Estragole, Ethyl Acetate, Ethyl Aceto Acetate, Ethyl Benzoate, Ethyl Heptoate, Ethyl Salicylate, Ethyl-2-Methyl Butyrate, Eucalyptol, Eugenol, Fenchyl Acetate, Fenchyl Alcohol, Methyl-2-octynoate, 2-sec-Butylcyclohexanone, Styralyl Acetate, Hexyl Acetate, lonone Alpha, Iso Amyl Acetate, Iso Butyl Acetate, Iso Menthone, Jasmone Cis, Laevo Carvone, Linalool, Linalool Oxide, Melonal, Menthol, Menthone, Methyl Acetophenone, Methyl Amyl Ketone, Methyl Benzoate, Methyl Heptenone, Methyl Hexyl Ketone, Methyl Para Cresol, Methyl Phenyl Acetate, Methyl Salicylate, Neral, Nerol, Para Cresol, Para Cresyl Acetate, Para Tolyl Aldehyde, Phenyl Acetaldehyde, Phenyl Ethyl Acetate, Phenyl Ethyl Butyrate, Phenyl Ethyl Formate, Phenyl Ethyl Iso Butyrate, Phenyl Ethyl Propionate, Phenyl Propyl Acetate, Phenyl Propyl Aldehyde, 4-Methyl-2-(2-methyl-1-propenyl)tetrahydropyran, Styralyl Propionate, Terpineol, Terpinolene, Trans-2-Hexenal, Hexyl Cinnamic Aldehyde Alpha, Oxacycloheptadec-10-en-2-one, Linalyl Benzoate, Cedrol, Benzyl Cinnamate, Linalyl Cinnamate, Phenyl Ethyl Cinnamate, Para Cresyl Phenyl Acetate, Benzyl Salicylate, Hexyl Salicylate, Phenyl Ethyl Salicylate, and Oxacyclohexadecan-2-one.

The edible film formulation of the present invention is particularly suitable for delivering flavourants containing aldehyde functionality, that are found in flavours such as cinnamon and artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Examples of aldehyde flavorings and their respective flavourant properties are acetaldehyde (apple); benzaldehyde (cherry, almond); cinnamic aldehyde (cinnamon); citral, i.e., alpha citral (lemon, lime); neral, i.e. beta citral (lemon, lime); decanal (orange, lemon); ethyl vanillin (vanilla, cream); heliotropine, i.e., piperonal (vanilla, cream); vanillin (vanilla, cream); alpha-amyl cinnamaldehyde (spicy fruity flavors); butyraldehyde (butter, cheese); valeraldehyde (butter, cheese); citronellal (modifies, many types); decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyl-5-heptenal, i.e. melonal (melon); 2-6-dimethyloctanal (green fruit); and 2-dodecenal (citrus, mandarin); cherry; grape; mixtures thereof; and the like.

The possibility of encapsulating aldehyde flavourants is a particular characteristic of the invention. These materials are capable of cross-linking certain useful film-forming materials such as the polypeptides, e.g. gelatin which affect the hydration time of the film such that it does not easily disperse or dissolve. By sequestering these aldehydes in a microcapsule, the loading of these materials may remain high and the properties of the film-forming materials may be preserved.

The amount of flavoring employed is normally a matter of preference subject to such factors as flavor type; individual flavor, and strength desired. Thus, the amount may be varied in order to obtain the result desired in the final product. Such variations are within the capabilities of those skilled in the art without the need for undue experimentation. In general, amounts of about 0.1 to about 30 wt % are useable with amounts of about 2 to about 25 wt % being preferred and amounts from about 8 to about 10 wt % are more preferred.

Food acids may be incorporated in the edible films to produce, a product that has a tartness or sourness, and to induce a mouth-watering effect when it is placed in the mouth. Food acids may be used to particularly useful effect in combination with citrus fruit flavours described herein above.

As food acids, there may be mentioned citric acid, malic acid, glacial acetic acid, anthranilic acid, tartaric acid, tiglic acid, ascorbic acid, benzoic acid, tannic acid, succinic acid, adipic acid, fumaric acid and lactic acid.

To achieve the tartness, sourness and salivation, food acids should be employed at levels greater than about 8% based on the dry weight of the edible film formulation, more particularly 8 to 20% dry weight. At these levels, the acids may adversely affect the properties of the film, for example the mechanical stability may be compromised and the hygroscopicity may increase. Accordingly, acids, or at least the majority of the acid may be usefully encapsulated in a microcapsule as described herein above.

Other organoleptic materials may be included as active agents, for example those coolant materials and sweetening agents commonly used in the art in confectionery, personal care products such as oral care or body care products and the like.

As pharmaceutical agents or nutraceutical agents may be mentioned agents that are intended to be placed in the oral cavity to administer a local effect, or to be absorbed across oral mucosa or open wounds to impart a local or systemic effect. Illustrative categories and representative examples include without limitation:-
(a) Antitussives, such as dextromethorphan, dextromethorphan hydrobromide, noscapine, carbetapentane citrate, and chlophedianol hydrochloride;
(b) Antihistamines, such as chlorpheniramine maleate, phenindamine tartrate, pyrilamione maleate, doxylamine succinate, and phenyltoloxamine citrate;
(c) Decongestants, such as phenylpherine hydrochloride, phenylpropanolamine hydrochloride, pseudoephedrine, hydrochloride ephedrine;
(d) Various alkaloids, such as codeine phosphate, codeine sulfate and morphine;
(e) Mineral supplements such as potassium chloride and calcium carbonates, magnesium oxide and other alkali metal and alkaline earth metal salts;
(f) Laxatives, vitamins and antacids;
(g) Ion exchange resins such as cholestyramine;
(h) Anti-cholesterolemic and anti-lipid agents such as gemfibrozil;
(i) Antiarrhythmics such as N-acetyl-procainamide;
(j) Antipyretics such as acetominophen, aspirin and ibuprofen;
(k) Appetite suppressants such as phenylpropanolamine hydrochloride or caffeine; and
(l) Expectorants such as quaifenesin.

Additional useful active medicaments include antiinflammatory substances, coronary dilators, cerebral dilators, peripheral vasodilators, anti-infectives, psychotropics, antimanics, stimulants, gastro-intestinal sedatives, antidiarrheal preparations, anti-anginal drugs, vasodilators, anti-hypertensive drugs, vasoconstrictors and migraine treatments, antibiotics, tranquilizers, antiphychotics, antitumor drugs, anticoagulants and antithrombotic drugs, hypnotics, sedatives, antiemetics, anti-nauseants, anticonvulsants, neuromuscular drugs, hyper- and hypoglycaemic agents, thyroid and antithyroid preparations, diuretics, antispasmodics, uterine relaxants, nutritional additives, antiobesity drugs, anabolic drugs, erythropoietic drugs, antiasthmatics, cough suppressants, mucolytics, anti-uricemic drugs, and the like. Mixtures of the drugs and medicaments may also be used.

The amount of pharmaceutical or nutraceutical agent employed will depend upon the particular condition to be treated and the particular active agent employed as will be appreciated by the skilled person.

The compositions of this invention may also contain coloring agents. The coloring agents are used in amounts effective to produce the desired color. The coloring agents useful in the present invention, include pigments such as titanium dioxide, which may be incorporated in amounts of up to about 5 wt %, and preferably less than about 1 wt %. Colorants can also include natural food colors and dyes suitable for food, drug and cosmetic applications. These colorants are known as FD&C dyes and lakes. The materials acceptable for the foregoing spectrum of use are preferably water-soluble, and include FD&C Blue No. 2, which is the disodium salt of 5,5-indigotindisulfonic acid. Similarly, the dye known as Green No. 3 comprises a triphenylmethane dye and is the monosodium salt of 4-[4-N-ethyl-p-sulfobenzylamino) diphenyl-methylene]-[1-N-ethyl-N-p-sulfonium benzyl)-2,5-cyclo-hexadienimine]. A full recitation of all FD&C and D&C dyes and their corresponding chemical structures may be found in the Kirk-Othmer Encyclopedia of Chemical Technology, Volume 5, Pages 857-884, which text is accordingly incorporated herein by reference.

Emulsifiers used in the present invention are optional ingredients that are advantageously employed when films are made by casting onto release papers. They may be selected from the group consisiting of lecithin, stearates, ester derivatives of stearates, palmitates, ester derivatives of palmitates, oleates, ester derivatives of oleates, glycerides, ester derivatives of glycerides, sucrose polyesters, polyglycerolesters, and animal waxes, vegetable waxes, synthetic waxes, petroleum , and mixtures thereof. Particularly useful emulsifiers are lecithin, non-ionic surfactants, such as polyoxyethylene sorbitan fatty acid esters, polyoxyethylene alkyl ethers, or polyoxyethylene castor oil derivatives with one or more polyalcohols, or mixtures thereof.

Emulsifiers may be employed in amounts of up to 2% by weight, more preferably up to 1% by weight.

Plasticisers may be employed in edible film compositions to impart flexibility to the film thereby to increase the ease of handling of the film during storage and during use. As plasticisers there may be mentioned any of those materials commonly used as plasticisers in edible film technology, in particular polyhydric alcohols such as glycerol, polyethylene glycol, propylene glycol, gycerin, sorbitol, maltitol and mannitol.

Plasticisers may be employed up to 5%, more preferably up to 1 % by weight.

The edible film as hereinabove described may be prepared according to a process comprising the steps of preparing an aqueous solution of hydrocolloid film-forming materials, and other optional ingredients as hereinabove described; mixing the solution until homogenous; adding microcapsules comprising active agent; casting the resultant mixture onto a releasable backing media; coating the mixture, for example using conventional knife-coating techniques; and drying the film. Further details as to the preferred process conditions of the present invention are set forth in the examples.

The drying operation may be carried out in a high-temperature air-bath, drying tunnel, vacuum drier, or any other suitable method. Because the films comprise microencapsulated active agents, even if those agents are thermally sensitive, relatively high temperatures may be employed, e.g. up to 90°C to reduce processing time, without substantially affecting the retention of the active agent or its integrity.

The edible film of the present invention may have a papery, wafer-like consistency that is possessed of sufficient mechanical strength to be handled without special precautions. The film may be provided in continuous sheets that may be rolled onto spools, or cut into sheets and stacked for storage. The films may be cut into any desirable shape for the particular intended end use, and packed in suitable containers.

The thickness of the films can be precisely controlled during the manufacturing process to vary, for example between 5 and 200 microns. The film may be a mono- or multi layer construction. In the case of a monolayer film, the microcapsules are dispersed throughout a monolayer of the hydrocolloid film-forming material. If the edible film is in the form of a multilayer, it may comprise a discrete layer consisting of the microcapsules, in addition to the layer of hydrocolloid film-forming material. The discrete layer may be formed according to any suitable process, e.g. microparticles may be sprayed or sprinkled onto a wet film before it passes through a drying process.

When placed directly in the mouth, the edible film is quickly hydrated and is softened and develops mucoadhesive properties; thereafter it disperses or dissolves rapidly in the oral cavity and so does not feel obtrusive or leave an unpleasant mouth feel. It is characteristic of the edible films that despite their dissolving or dispersing rapidly, the microparticles linger in the oral cavity so creating a prolonged release of active agent without attendant adverse mouth feel. One is therefore able to effect a long-lasting taste, or breath-freshening sensation, e.g. for 20 minutes or more. Alternatively, or in addition, one can achieve a prolonged release of a pharmaceutical active either locally or systemically. In existing commercial products, once the film has dissolved such that there is no longer any unpleasant mouth feel, the flavour sensation or the cosmetic or pharmaceutical effect is lost relatively rapidly thereafter as the active agent is quickly washed away by saliva. The microcapsules, in contrast, are retained in the oral cavity for longer time periods by being physically trapped in pits or fissures in the oral tissue, or by possessing certain mucoadhesive properties similar to those of the film.

Despite the use of relatively high loading of active agent and the use of film ingredients that may attract moisture, e.g. certain plasticisers may also have humectant properties, the films are surprisingly mechanically robust and hygroscopically stable. For example, edible film may contain more than about 25% of flavour oils, and still be resistant to self-adhesion under stress conditions (e.g. 30°C/85% r.h.) for periods of up to 4 days or more. These results compare favourably with commercially available edible films, that even contain less flavour. Accordingly, edible films of the present invention may have considerably improved shelf-life, and are easier to store and handle than known edible films. This hygroscopic stability is in some part the result of sequestering active agent, e.g. flavour oils, in microcapsules, however, without intending to bound by any particular theory, applicant also believes that microcapsules disposed within the film present themselves at the surface of the film, affecting the surface activity of the film thereby to impart a non-tacky feel to the film. Of course, if an additional layer of microparticles is added film, this additional layer will present a non-tacky surface.

The present invention has been described thus far relation to active agent-containing films intended for presentation on food or directly in the mouth. However, the skilled person will appreciate that one may encapsulate active agents and add the microcapsules to film-forming compositions for all manner of applications. Accordingly, it is within the scope of the invention to provide fragranced films or to provide films that may contain moisturising materials that may be placed on the skin of an end user, to impart fragrance and/or moisturising effects. Furthermore, the film may be deposited on substrates that are intended to be stored under dry conditions, but in use are exposed to liquids. For example, edible film may be coated on a surface, e.g. inner surface of a straw for soft drinks or beverages. The film may contain an flavour ingredient, e.g. citral that is unstable in the beverage, e.g. a carbonated beverage, but may be admixed with the beverage as it is drawn up the straw by the consumer. Another embodiment of this application is contemplated wherein the edible film is contained in a bottle cap such that the film is isolated from the beverage by means of a frangible seal placed across the cap, which frangible seal is broken when the cap is opened by a consumer, to permit the film to drop into the beverage and rapidily dissolve therein. The present invention therefore permits of the mixing of incompatible flavourants into foods and beverages to present a desired flavour accord, without the need for the consumer to conciously have to mix together the ingredients.

There now follows a series of examples that serve to illustrate the invention.
Figure 1: compares graphically flavour intensity and flavour character of a film prepared by addition of flavour components directly to a film (414-09000-13) and a film prepared by adding microcapsulated flavour components (414-09000-14).
Figure 2: compares the hygroscopic stability of a film of the present invention and commercially available films in which flavour components are added directly to the film.
Figure 3: Compares the Residual Moisture at 30°C and 85% r.h.of a film of the present invention and two commerically available films in which flavour components are added directly to the film.

### Example I

A mono-layer hydrocolloid matrix containing encapsulated flavouring agents was prepared as follows:

### Encapsulated Flavouring Agents:

A solution was made by adding 2.0 grams of Menthol Carboxamide and 5.0 grams deionized water into 40 grams Cinnamon Oil. This solution was mixed with 53.0 grams of Flavorburst ® Dry Protein Encapsulate (Givaudan) for 30 minutes. Flavouring agents were absorbed into Flavorburst ® after 30 minutes and a dry encapsulated powder was formed.

### Polymer Solutions :

### Starch Solution

10 grams of Ultra-Spersece® A and 10 grams Textra ® Plus were added to 180 ml of deionized water with high shear mixing until a clear solution was formed.

### Protein Solution

Heat 70 ml of deionized water to 40°C. Slowly add 30 grams of Fish gelatin mix with slow agitation until gelatin is dissolved. Cool to 30°C and maintain temperature.

### Coating Solution :

| | |
|---|---|
| Starch Solution | 53 grams |
| Protein Solution | 35 grams |
| Sorbo Sorbitol Solution | 2.2 grams |
| Polysorbate 80 | 0.8 grams |
| Encapsulated Flavouring Agents | 9.0 grams |

Add Starch Solution, Sorbo Sorbitol Solution and Polysorbate 80 until homogeneous. Add Protein Solution and mix until homogeneous. Add Encapsulated Flavour Agents and mix until no lumps are present.

### Coating:

Coating solution was coated onto a polyethylene coated differential release paper using a knife-over-roll coating head. The coated paper was then dried in a drying tunnel and hydrocolloid matrix was formed. Hydrocolloid matrix has a paper wafer like consistency. The hydrocolloid matrix with encapsulated flavouring agents was then cut into pieces. Pieces were then tested for sensory response of flavour release in the oral cavity. The films dissolved quickly in the mouth to provide sustained bursts of flavour, cooling and tingling sensations to the oral cavity.

### Example II

A mono-layer hydrocolloid matrix containing encapsulated flavouring agents was prepared as follows :

### Encapsulated Flavouring Agents :

A solution was made by adding 2.0 grams of Menthol Carboxamide and 5.0 grams deionized water into 40 grams Peppermint Oil . This solution was mixed with 53.0 grams of Flavorburst ® Dry Protein Encapsulate for 30 minutes. Flavouring agents were absorbed into Flavorburst ® after 30 minutes and a dry encapsulated powder was formed.

### Polymer Solutions:

### Starch Solution

10 grams of Ultra-Sperse® A and 10 grams Textra ® Plus were added to 180 ml of deionized water with high shear mixing until a clear solution was formed.

### Protein Solution

Heat 70 ml of deionized water to 40°C. Slowly add 30 grams of Fish gelatin mix with slow agitation until gelatin is dissolved. Cool to 30°C and maintain temperature.

### Coating Solution :

| | |
|---|---|
| Starch Solution | 44 grams |
| Protein Solution | 44 grams |
| Sorbo Sorbitol Solution | 2.2 grams |
| Polysorbate 80 | 0.8 grams |
| Encapsulated Flavouring Agents | 9.0 grams |

Add Starch Solution, Sorbo Sorbitol Solution and Polysorbate 80 until homogeneous. Add Protein Solution and mix until homogeneous. Add Encapsulated Flavour Agents and mix until no lumps are present.

### Coating :

Coating solution was coated onto a polyethylene coated differential release paper using a knife-over-roll coating head. The coated paper was then dried in a drying tunnel and hydrocolloid matrix was formed. Hydrocolloid matrix has a paper wafer like consistency. The hydrocolloid matrix with encapsulated flavouring agents was then cut into pieces. Pieces were then tested for sensory response of flavour release in the oral cavity. The films dissolved quickly in the mouth to provide sustained bursts of flavour, cooling and tingling sensations to the oral cavity.

### Example III

A multi layer hydrocolloid matrix containing encapsulated flavouring agents was prepared as follows :

### Encapsulated Flavouring Agents:

A solution was made by adding 2.0 grams of Menthol Carboxamide and 5.0 grams deionized water into 40 grams Cinnamon Oil . This solution was mixed with 53.0 grams of Flavorburst ® Dry Protein Encapsulate for 30 minutes. Flavouring agents were absorbed into Flavorburst ® after 30 minutes and a dry encapsulated powder was formed.

### Polymer Solutions:

### Starch Solution

10 grams of Ultra-Sperse® A and 10 grams Textra ® Plus were added to 180 ml of deionized water with high shear mixing until a clear solution was formed.

### Protein Solution

Heat 70 ml of deionized water to 40°C. Slowly add 30 grams of Fish gelatin mix with slow agitation until gelatin is dissolved. Cool to 30°C and maintain temperature.

### Coating Solution :

| | |
|---|---|
| Starch Solution | 48 grams |
| Protein Solution | 48 grams |
| Sorbo Sorbitol Solution | 3.0 grams |
| Polysorbate 80 | 1.0 grams |

Add Starch Solution, Sorbo Sorbitol Solution and Polysorbate 80 until homogeneous. Add Protein Solution and mix until homogeneous.

### Coating :

Coating solution was coated onto a polyethylene coated differential release paper using a knife-over-roll coating head. Once coating solution had been applied to differential release paper, encapsulated flavouring agents were applied topically to the coating. The coated paper was then dried in a drying tunnel and hydrocolloid matrix was formed. Hydrocolloid matrix has a paper wafer-like consistency. The hydrocolloid matrix with encapsulated flavouring agents was then cut into pieces. Pieces were then tested for sensory response of flavour release in the oral cavity. The films dissolved quickly in the mouth to provide sustained bursts of flavour, cooling and tingling sensations to the oral cavity.

### Example IV

A formulation containing fruit flavours and food acid was formed according to the aforementioned methodology.

| | Wet Wt | Dry Wt |
|---|---|---|
| Deionised Water | 582.7 | |
| Pure Coat 792 Modified Starch | 20 | 20 |
| HPMC | 35 | 35 |
| Gelatin | 97 | 97 |
| Polysorbate 80 | 10 | 10 |
| Glycerine | 20 | 20 |
| Sodium Saccharine | 5 | 5 |
| FDC Red 40 Lake | 0.3 | 0.3 |
| Malic acid | 50 | 50 |
| Cherry Emulsion | 130 | 48.1 |
| Cherry Encapsulated | 50 | 50 |
| TOTAL | 1000 | 335.4 |

The edible film produce was papery, wafer-like in consistency, dry to the touch and capable of being stored in adjacent layers without sticking. When presented to the mouth it imparted an immediate mouth-watering sensation and flavour with the flavour lasting for a period of up to 20 minutes.

### Example V

This example compares the flavour characteristics of two films. In a first film, the flavour material is added directly to film forming ingredients as an oil. In a second film, the flavour material is microencapsulated and the microcapsules are then added to the other film ingredients.

### Flavour Oil in Film Matrix

| Coating Solution I: | |
|---|---|
| Starch Solution | 44 grams |
| Protein Solution | 44 grams |
| Sorbo Sorbitol Solution | 2.2 grams |
| Polysorbate 80 | 0.8 grams |
| Cinnamon Oil | 4.0 grams |

Add Starch Solution, Sorbo Sorbitol Solution, Cinnamon Oil and Polysorbate 80 until homogeneous. Add Protein Solution and mix until homogeneous.

### Coating :

Coating solution was coated onto a polyethylene coated differential release paper using a knife-over-roll coating head. The coating was then dried in a drying tunnel at 170°F and flavour containing film was formed. The flavour containing film was then cut into pieces. Pieces were then tested by Gas Chromatography (GC) for flavour oil content in the film matrix.

### Encapsulated Flavouring in Film Matrix

### Encapsulated Flavouring Agents:

A solution was made by adding 2.0 grams of Menthol Carboxamide and 5.0 grams deionized water into 40 grams Cinnamon Oil . This solution was mixed with 53.0 grams of Flavorburst ® Dry Protein Encapsulate for 30 minutes. Flavouring agents were absorbed into Flavorburst ® after 30 minutes and a dry encapsulated powder was formed.

### Polymer Solutions :

### Starch Solution

10 grams of Ultra-Sperse®A and 10 grams Textra ® Plus were added to 180 ml of deionized water with high shear mixing until a clear solution was formed.

### Protein Solution

Heat 70 ml of deionized water to 40°C. Slowly add 30 grams of Fish gelatin mix with slow agitation until gelatin is dissolved. Cool to 30°C and maintain temperature.

### Coating Solution :

| | |
|---|---|
| Starch Solution | 43 grams |
| Protein Solution | 43 grams |
| Sorbo Sorbitol Solution | 3.0 grams |
| Polysorbate 80 | 1.0 grams |
| Encapsulated Cinnamon Flavour | 10 grams |

Add Starch Solution, Sorbo Sorbitol Solution and Polysorbate 80 until homogeneous. Add Protein Solution and mix until homogeneous. Add Encapsulated Flavour Agents and mix until no lumps are present.

### Coating :

Coating solution was coated onto a polyethylene coated differential release paper using a knife-over-roll coating head. The coated paper was then dried in a drying tunnel, at 170°F, and hydrocolloid matrix was formed. Hydrocolloid matrix has a paper wafer like consistency. The hydrocolloid matrix with encapsulated flavouring agents was then cut into pieces. Pieces were then tested by GC for flavour oil content in the film matrix.

### Gas Chromatography (GC) Method

The sample extraction method used is as follows:
1.) Add and record weight of film (0.5000 - 1.0000 grams) to a centrifuge tube
2.) Add and record weight of 2-3 drops of Chlorocyclohexane (standard) to the tube. Remove from balance & add 10.0 ml of HPLC water
3.) Vortex mix until dissolved & allow to stand 1-2 hours to totally hydrate the film.
4.) Add HPLC grade Acetone and vortex mix for 1-2 minutes. Let sample stand to equilibrate (time varied above)
5.) Draw sample off from top and add to G.C. autosampler vial for sampling to the instrument (Hewlitt-Packard HP5890A with HP 7673 Autosampler).

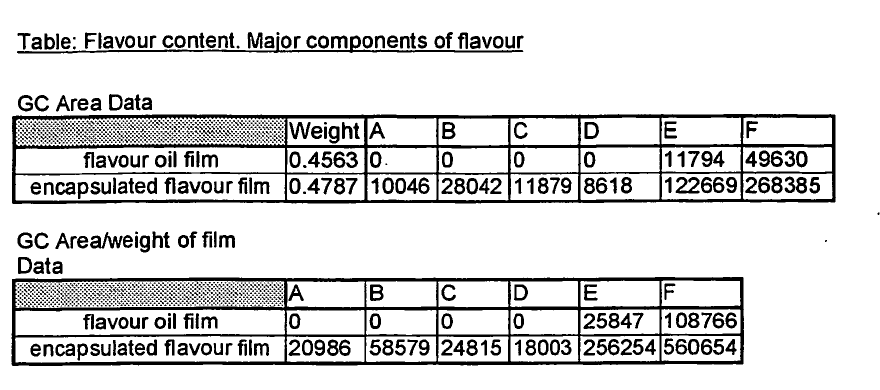

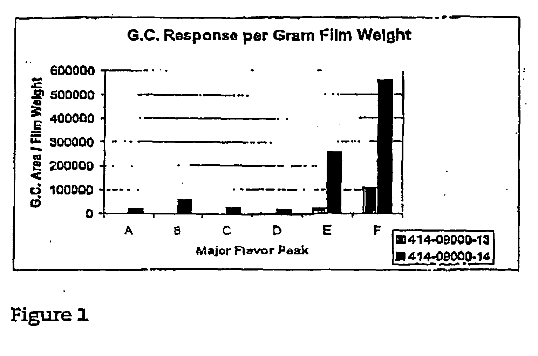

Wherein A through F are representative of characteristic peaks of the flavour material. The GC Area/Weight of film data is shown graphically in Figure 1 wherein "414-09000-13" represents the film wherein the flavour oil was added directly to the film, and "414-09000-14" represents a film wherein the flavour was added in microencapsulated form.

Microencapsulation of cinnamon flavouring agents provided thermal stability of volatile components during the drying process of the hydrocolloid matrix. Thus the profile of the flavouring agents is well preserved and an exceptional flavour profile was delivered. Incorporation of the flavour oil into the film matrix produced a film that had substantial flavour loss during the drying process, and the film product did not have same flavour profile of the original oil.

### Example VI

### Encapsulated Flavouring in Film Matrix

### Encapsulated Flavouring Agents:

A solution was made by adding 2.0 grams of Menthol Carboxamide and 5.0 grams deionized water into 40 grams Cinnamon Oil . This solution was mixed with 53.0 grams of Flavorburst ® Dry Protein Encapsulate for 30 minutes. Flavouring agents were absorbed into Flavorburst ® after 30 minutes and a dry encapsulated powder was formed.

### Polymer Solutions:

### Starch Solution

10 grams of Ultra-Sperse® A and 10 grams Textra ® Plus were added to 180 ml of deionized water with high shear mixing until a clear solution was formed.

### Protein Solution

Heat 70 ml of deionized water to 40°C. Slowly add 30 grams of Fish gelatin mix with slow agitation until gelatin is dissolved. Cool to 30°C and maintain temperature.

### Coating Solution :

| | |
|---|---|
| Starch Solution | 43 grams |
| Protein Solution | 43 grams |
| Sorbo Sorbitol Solution | 3.0 grams |
| Polysorbate 80 | 1.0 grams |
| Encapsulated Cinnamon Flavour | 10 grams |

Add Starch,Solution, Sorbo Sorbitol Solution and Polysorbate 80 until homogeneous. Add Protein Solution and mix until homogeneous. Add Encapsulated Flavour Agents and mix until no lumps are present.

### Coating :

Coating solution was coated onto a polyethylene coated differential release paper using a knife-over-roll coating head. The coated paper was then dried in a drying tunnel, at 170°F, and hydrocolloid matrix was formed. Hydrocolloid matrix has a paper wafer like consistency. The hydrocolloid matrix with encapsulated flavouring agents was then cut into pieces. Pieces were then tested for accelerated ageing at 30°C/85% RH.

### Hygroscopic Stability Testing

Film samples were placed in 30°C/85% Environmental Chamber. Film samples were stacked on top of each other and open to chamber environment. Samples were monitored every 4 hours for sticking and melting of film samples. Samples were evaluated with the following ranking system :
1 - Non sticking of films together
2 -Films sticking together, but can be pulled apart.
3 - Total sticking of adjacent films to leave a solid mass

### Hygroscopic Stability of Film Products at 30C/85% RH

| **Samples** | **4 hr** | **8 hr** | **16 hr** | **24 hr** | **48 hr** | **72 hr** | **96 hr** |
|---|---|---|---|---|---|---|---|
| Encapsulated Cinnamon Film | 1 | 1 | 1 | 1 | 1 | 2 | 3 |
| Walgreen's Fresh Breath | 3 | 3 | 3 | 3 | 3 | 3 | 3 |
| Strips | | | | | | | |
| Listerine® Film | 3 | 3 | 3 | 3 | 3 | 3 | 3 |

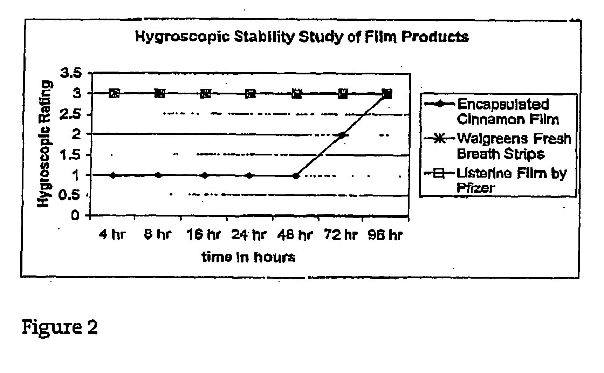

The commercially available films are extremely sensitive to heat and moisture. The commercial films tested melted into a solid mass after 4 hours at 30°C/85% RH. Encapsulated Cinnamon Film was stable at 30°C/85% RH. for 72 hours (see Figure 2).

### Residual Moisture of Film Products at 30°C/85% RH Stability Testing

Film samples were tested for residual moisture by Karl Fisher Titration. Samples were tested prior to be placed in environmental chamber, and every 24 hours in the chamber.

| **Samples** | **Residual Moisture by Karl Fischer** | | | | | |
|---|---|---|---|---|---|---|
| | **Day 0** | **24 Hours** | **48 Hours** | **72 Hours** | **96 Hours** | **120 Hours** |
| Encapsulated Cinnamon | 11.51 | 15.65 | 16.71 | 17.12 | 17.67 | 18.43 |
| Film | | | | | | |
| Walgreen's Fresh | 9.82 | 20.45 | 25.89 | 35.92 | | |
| Breath Strips | | | | | | |
| Listerine® Film | 10.11 | 20.99 | 27.89 | 33.98 | | |

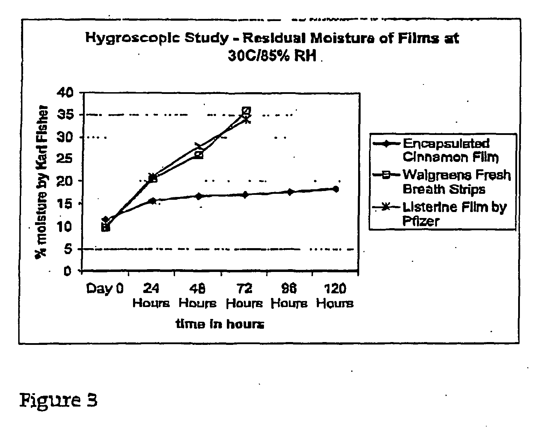

The commercially available films tested absorbed moisture continually during storage at 30°C/85%RH. Encapsulated Cinnamon Film absorbed moisture during first 24 hours, but at a much lower rate than commercially available films and reaches a state of equilibrium for moisture absorption (see Figure 3).

## Claims

1. An edible film wherein said film comprises a hydrocolloid film-fonning material having dispersed therein a plurality of microcapsules containing an active agent.

2. An edible film according to claim 1 wherein the microcapsules comprise a first population of microcapsules containing one active agent, and a second population of microcapsules contain a second active agent, different from the first active agent.

3. An edible film according to claim 1 wherein the active agent is selected from the group consisting of flavourants, fragrances, pharmceuticals nutraceuticals, or food acids.

4. An edible film according to claim 1 wherein microcapsules contain a colourant.

5. An edible film according to claim 2 wherein a first population of microcapsules contain a flavourant, and a second population contain a food acid.

6. An edible film according to claim 5 wherein the flavourant is a fruit flavour, and the food acid is selected from the group consisting of citric acid, malic acid, glacial acetic acid, anthranilic acid, tartaric acid, tiglic acid, ascorbic acid, benzoic acid, tannic acid, succinic acid, adipic acid, fumaric acid and lactic acid.

7. An edible film according to claim 2 wherein a first population of microcapsules contains a pharmaceutical agent, and a second population contains a flavourant.

8. An edible film according to claim 1 wherein an active agent is in admixture with the hydrocolloid film-forming material.

9. An edible film according to claim 1 wherein the microcapsule consists of a hydrogel shell and an core of active agent.

10. An edible film according to claim 1 wherein the microencapsulating material is selected from the group consisting of polymeric carbohydrates or water-soluble polypeptides, such as one or more materials selected from starches; chemically modified starches; alginates, e.g. sodium alginate; pectins; tragacantha, acacia gum, gum arabic; agar; carrageenan; gelatin; casein; soy protein; whey protein; cellulose polymers such as hydroxypropylmethyl cellulose, hydroxyethyl cellulose or hydroxypropyl cellulose, or mixtures thereof; wheat glutten, furcellaran, locust bean gums, chitosan, xanthan gum, gellan gum, pullalan and mixtures thereof; synthetic polymers that are soluble or swellable in water, such as polyvinylpyrrolidone, polyvinylalcohol, polyacrylic acid, or polyacrylamide.

11. A film according to claim 10 wherein the protein is a gelatin.

12. A film according to claim 10 wherein the carbohydrate is alginate.

13. A film according to claim 1 additionally comprising one or more ingredients selected from the group consisting of a disintegrant, an emulsifier, and a plasticiser.

14. A film according to claim 13 wherein the disintegrant is selected from the group consisting of water-soluble or water dispersible ingredients selected from the group consisting of a sugar, such as lactose, glucose, or mannose; a sugar alcohol, such as mannitol, sorbitol, xylitol, erythritol, lactitol, or maltitol; corn starch, potato starch, rice starch, tapioca starch, maize starch or sodium starch glycolate; synthetic polymers selected from polyethylene glycol, polyvinylpyrrolidone, polyvinylalcohol, polyoxyethylene copolymers, polyoxypropylene copolymers, or polyethyleneoxide; cellulose-based materials selected from modified cellulose, crosscarmellose sodium and microcrystalline cellulose or a mixture of any of these compounds. ,

15. A film according to claim 13 wherein the emulsifier is selected from the group consisting of lecithin, stearates, ester derivatives of stearates, palmitates, ester derivatives of palmitates, oleates, ester derivatives of oleates, glycerides, ester derivatives of glycerides, sucrose polyesters, polyglycerolesters, and animal waxes, vegetable waxes, synthetic waxes, petroleum, non-ionic surfactants, selected from polyoxyethylene sorbitan fatty acid esters, polyoxyethylene alkyl ethers, or polyoxyethylene castor oil derivatives with one or more polyalcohols, or mixtures of any of the above.

16. A film according to claim 13 wherein the plasticiser is a polyhydric alcohol selected from the group consisting of glycerol, polyethylene glycol, propylene glycol, gycerin, sorbitol, maltitol and mannitol.

17. An edible film according to claim 1 in the form of a monolayer.

18. A package comprising a plurality of strips of an edible film according to claim 1

## Patentansprüche

1. Essbarer Film, worin dieser Film umfasst ein hydrokolloides Film bildendes Material, in welchem eine Pluralität an Mikrokapseln dispergiert ist, die einen Wirkstoff enthalten.

2. Essbarer Film nach Anspruch 1, worin die Mikrokapseln umfassen eine erste Population an Mikrokapseln, die einen Wirkstoff enthalten, und eine zweite Population an Mikrokapseln, die einen Wirkstoff enthalten, der vom ersten Wirkstoff verschieden ist.

3. Essbarer Film nach Anspruch 1, worin der Wirkstoff aus der Gruppe ausgewählt ist, die besteht aus Geschmacksstoffen, Duftstoffen, Pharmazeutika, Nutrazeutika oder Nahrungsmittelsäuren.

4. Essbarer Film nach Anspruch 1, worin die Mikrokapseln einen Farbstoff enthalten.

5. Essbarer Film nach Anspruch 2, worin eine erste Population an Mikrokapseln einen Geschmacksstoff enthält und eine zweite Population eine Nahrungsmittelsäure enthält.

6. Essbarer Film nach Anspruch 5, worin der Geschmacksstoff ein Fruchtgeschmack ist und die Nahrungsmittelsäure aus der Gruppe ausgewählt ist, die besteht aus Citronensäure, Apfelsäure, Eisessigsäure, Anthranilsäure, Weinsäure, Tiglinsäure, Ascorbinsäure, Benzoesäure, Tanninsäure, Bernsteinsäure, Adipinsäure, Fumarsäure und Milchsäure.

7. Essbarer Film nach Anspruch 2, worin eine erste Population von Mikrokapseln ein pharmazeutisches Mittel enthält und eine zweite Population einen Geschmacksstoff enthält.

8. Essbarer Film nach Anspruch 1, worin ein Wirkstoff im Gemisch mit dem hydrokolloiden Film bildenden Material vorliegt.

9. Essbarer Film nach Anspruch 1, worin die Mikrokapsel aus einer Hydrogelschale und einem Wirkstoff-Kern besteht.

10. Essbarer Film nach Anspruch 1, worin das Material zur Mikroeinkapselung aus der Gruppe ausgewählt ist, die besteht aus polymeren Kohlenhydraten oder wasserlöslichen Polypeptiden, wie ein oder mehr Materialien, die ausgewählt sind aus Stärken, chemisch modifizierten Stärken, Alginaten, beispielsweise Natriumalginat, Pektinen, Tragacanth, Akaziengummi, Gummi arabicum, Agar, Karrageenan, Gelatine, Casein, Sojaprotein, Molkeprotein, Cellulosepolymeren, wie Hydroxypropylmethylcellulose, Hydroxyethylcellulose oder Hydroxypropylcellulose, oder Gemischen hiervon, Weizengluten, Furcellaran, Johannisbrotkernmehle, Chitosan, Xanthangummi, Gellangummi, Pullalan, und Gemischen hiervon, synthetischen Polymeren, die in Wasser löslich oder quellbar sind, wie Polyvinylpyrrolidon, Polyvinylalkohol, Polyacrylsäure oder Polyacrylamid.

11. Film nach Anspruch 10, worin das Protein eine Gelatine ist.

12. Film nach Anspruch 10, worin das Kohlenhydrat ein Alginat ist.

13. Film nach Anspruch 1, zusätzlich umfassend ein oder mehr Ingredientien, die aus der Gruppe ausgewählt sind, die besteht aus einem Zerfallhilfsmittel, einem Emulgator und einem Weichmacher.

14. Film nach Anspruch 13, worin das Zerfallhilfsmittel aus der Gruppe ausgewählt ist, die besteht aus in Wasser löslichen oder in Wasser dispergierbaren Ingredientien, die aus der Gruppe ausgewählt sind, die besteht aus einem Zucker, wie Lactose, Glucose oder Mannose, einem Zuckeralkohol, wie Mannit, Sorbit, Xyllit, Erythrit, Lactit oder Maltitol, Getreidestärke, Kartoffelstärke, Reisstärke, Tapiokastärke, Maisstärke oder Natriumstärkeglycolat, synthetischen Polymeren, die ausgewählt sind aus Polyethylenglycol, Polyvinylpyrrolidon, Polyvinylalkohol, Polyoxyethylencopolymeren, Polyoxypropylencopolymeren oder Polyethylenoxid, Materialien auf Basis von Cellulose, die ausgewählt sind aus modifizierter Cellulose, Croscarmellosenatrium und mikrokristalliner Cellulose, oder einem Gemisch dieser Verbindungen.

15. Film nach Anspruch 13, worin der Emulgator aus der Gruppe ausgewählt ist, die besteht aus Lecithin, Stearaten, Esterderivaten von Stearaten, Palmitaten, Esterderivaten von Palmitaten, Oleaten, Esterderivaten von Oleaten, Glyceriden, Esterderivaten von Glyceriden, Saccharosepolyestern, Polyglycerinestern und tierischen Wachsen, pflanzlichen Wachsen, synthetischen Wachsen, Petroleumwachs, nicht-ionischen Tensiden, die ausgewählt sind aus Polyoxyethylensorbitanfettsäureestern, Polyoxyethylenalkylethern oder Polyoxyethylenrizinusölderivaten mit ein oder mehr Polyalkoholen oder Gemischen der obigen Substanzen.

16. Film nach Anspruch 13, worin der Weichmacher ein Polyhydroxyalkohol ist, der aus der Gruppe ausgewählt ist, die besteht aus Glycerol, Polyethylenglycol, Propylenglycol, Glycerin, Sorbit, Maltit und Mannit.

17. Essbarer Film nach Anspruch 1 in Form einer Monoschicht.

18. Packung, umfassend eine Pluralität an Streifen eines essbaren Films nach Anspruch 1.

## Revendications

1. Film comestible, dans lequel ledit film comprend un matériau filmogène hydrocolloïdal contenant à l'état dispersé une pluralité de microcapsules contenant un agent actif.

2. Film comestible selon la revendication 1, dans lequel les microcapsules comprennent une première population de microcapsules contenant un agent actif, et une deuxième population de microcapsules contient un deuxième agent actif, différent du premier agent actif.

3. Film comestible selon la revendication 1, dans lequel l'agent actif est choisi dans le groupe constitué par des agents aromatisants, des fragrances, des agents nutritifs pharmaceutiques ou des acides alimentaires.

4. Film comestible selon la revendication 1, dans lequel les microcapsules contiennent un colorant.

5. Film comestible selon la revendication 2, dans lequel une première population de microcapsules contient un agent aromatisant, et une deuxième population de microcapsules contient un acide alimentaire.

6. Film comestible selon la revendication 5, dans lequel l'agent aromatisant est un arôme de fruit et l'acide alimentaire est choisi dans le groupe constitué par l'acide citrique, l'acide malique, l'acide acétique glacial, l'acide anthranilique, l'acide tartrique, l'acide tiglique, l'acide ascorbique, l'acide benzoïque, l'acide tannique, l'acide succinique, l'acide adipique, l'acide fumarique et l'acide lactique.

7. Film comestible selon la revendication 2, dans lequel une première population de microcapsules contient un agent pharmaceutique et une deuxième population contient un agent aromatisant.

8. Film comestible selon la revendication 1, dans lequel un agent actif est mélangé avec le matériau filmogène hydrocolloïdal.

9. Film comestible selon la revendication 1, dans lequel la microcapsule est constituée d'une enveloppe d'hydrogel et d'un noyau d'agent actif.

10. Film comestible selon la revendication 1, dans lequel le matériau de microencapsulation est choisi dans le groupe constitué par des hydrates de carbone polymères et des polypeptides hydrosolubles, tels qu'un un plusieurs matériaux choisis parmi des amidons, des amidons chimiquement modifiés ; des alginates, par exemple l'alginate de sodium ; des pectines ; la gomme adragante, la gomme d'acacia, la gomme arabique ; la gélose ; la carraghénine ; la gélatine ; la caséine ; une protéine de soja ; une protéine de petit-lait ; des polymères cellulosiques tels que l'hydroxypropylméthylcellulose, l'hydroxyéthylcellulose ou l'hydroxypropylcellulose, ou leurs mélanges ; le gluten de blé, la furcellarane, des gommes de caroube, le chitosan, la gomme de xanthane, la gomme de gellane, la pullulane et leurs mélanges ; des polymères synthétiques qui sont hydrosolubles ou peuvent gonfler dans l'eau, tels que la polyvinylpyrrolidone, l'alcool polyvinylique, l'acide polyacrylique ou le polyacrylamide.

11. Film selon la revendication 10, dans lequel la protéine est une gélatine.

12. Film selon la revendication 10, dans lequel l'hydrate de carbone est un alginate.

13. Film selon la revendication 1, comprenant en outre un ou plusieurs ingrédients choisis dans le groupe constitué par un agent de désintégration, un émulsionnant et un plastifiant.

14. Film selon la revendication 13, dans lequel l'agent de désintégration est choisi dans le groupe constitué par des ingrédients solubles ou dispersibles dans l'eau choisis dans le groupe constitué par un sucre, tel que le lactose, le glucose ou le mannose ; un alcool de sucre, tel que le mannitol, le sorbitol, le xylitol, l'érythritol, le lactitol ou le maltitol ; l'amidon de maïs, l'amidon de pomme de terre, l'amidon de riz, la fécule de manioc, la fécule de maïs ou le glycolate d'amidon sodique ; des polymères synthétiques choisis parmi le polyéthylèneglycol, la polyvinylpyrrolidone, l'alcool polyvinylique, des copolymères de polyoxyéthylène, des copolymères de polyoxypropylène, ou l'oxyde de polyéthylène ; des matériaux à base de cellulose choisis parmi la cellulose modifiée, la croscarmellose sodique et la cellulose microcristalline ou un mélange de n'importe lesquels de ces composés.

15. Film selon la revendication 13, dans lequel l'émulsionnant est choisi dans le groupe constitué par la lécithine, des stéarates, des dérivés esters de stéarates, des palmitates, des dérivés esters de palmitate, des oléates, des dérivés esters d'oléates, des glycérides, des dérivés esters de glycérides, des polyesters de saccharose, des polyglycolesters, et des cires animales, des cires végétales, des cires synthétiques, le petroleum, des agents tensioactifs non ioniques choisis entre des esters d'acides gras de polyoxyéthylènesorbitan, des éthers alkyliques de polyoxyéthylène et des dérivés de polyoxyéthylène-huile de ricin avec un ou plusieurs polyalcools ou des mélanges de n'importe lesquels des composés ci-dessus.

16. Film selon la revendication 13, dans lequel le plastifiant est un alcool polyhydrique choisi dans le groupe constitué par le glycérol, le polyéthylèneglycol, le propylèneglycol, la glycérine, le sorbitol, le maltitol et le mannitol.

17. Film comestible selon la revendication 1 sous forme d'une monocouche.

18. Conditionnement comprenant une pluralité de bandes d'un film comestible selon la revendication 1.
